# FASCICULE DE BREVET EUROPEEN

(11) **EP 4 240 434 B1**
(45) Date de publication et mention de la délivrance du brevet: **08.07.2026**
(21) Numéro de dépôt: 21802376.0
(22) Date de dépôt: 08.11.2021
(51) Int. Cl.: A61L 27/18, A61L 27/56, A61L 27/58, A61F 2/12

(54) **DISPOSITIF IMPLANTABLE, NOTAMMENT MAMMAIRE**
IMPLANTIERBARE VORRICHTUNG, INSBESONDERE IMPLANTIERBARE BRUSTVORRICHTUNG
IMPLANTABLE DEVICE, IN PARTICULAR AN IMPLANTABLE BREAST DEVICE

(30) Priorité: 09.11.2020 FR 2011492
(43) Date de publication de la demande: 13.09.2023
(73) Titulaire: LATTICE MEDICAL, 59120 Loos (FR)
(72) Inventeur: DESTOUESSE VILLA, Jaime Andres, 59800 LILLE (FR); CAO, Shengheng, 59000 LILLE (FR)
(74) Mandataire: Cabinet Beau de Loménie
(86) Numéro de dépôt international: PCT/EP2021/080987
(87) Numéro de publication internationale: WO 2022/096729

(56) Documents cités:
- WO-A1-2018/078489
- WO-A1-2019/238716
- US-A1- 2014 135 925
- FINDLAY MICHAEL W. ET AL: "Tissue-Engineered Breast Reconstruction : Bridging the Gap toward Large-Volume Tissue Engineering in Humans", vol. 128, no. 6, 1 December 2011 (2011-12-01), US, pages 1206 - 1215, XP055818975, ISSN: 0032-1052, Retrieved from the Internet <URL:http://dx.doi.org/10.1097/PRS.0b013e318230c5b2> DOI: 10.1097/PRS.0b013e318230c5b2

## Description

### Domaine Technique

La présente invention a pour objet un dispositif implantable, notamment mammaire, ainsi qu'un procédé de fabrication dudit dispositif.

La présente invention a pour objet un dispositif implantable pour remplacer et/ou augmenter un volume de tissu mou, notamment suite au retrait d'un volume de tissu du sujet malade.

### Etat de la technique

Un implant mammaire peut être implanté pour augmenter le volume d'un sein sans que le sein ne présente une pathologie particulière, ou en reconstruction de ce dernier suite à un retrait d'un volume donné de tissu malade.

Il existe de nombreux types d'implants mammaires à ce jour. Les implants mammaires formés d'une poche remplie d'un gel de silicone, ont pour inconvénients que la poche peut se rompre libérant alors le gel de silicone. La poche remplie de silicone est par ailleurs implantée de manière permanente dans le corps.

On connait des dispositifs implantables, décrits dans WO 2018/078489, comprenant une coque délimitant un volume déterminé dans lequel un tissu mou va croître, ladite coque recevant au moins un pédicule vasculaire et un volume de tissu mou, tel qu'un volume de tissu adipeux, disposé sur le pédicule vasculaire. Le pédicule vasculaire permet la vascularisation, et ainsi l'alimentation du tissu adipeux qui croît et remplit la coque. La coque comprend quelques ouvertures traversantes permettant d'alimenter le volume de tissu mou en liquide séreux riche en facteurs de croissance. Il est également prévu que les ouvertures traversantes latérales pour le placement d'un pédicule soient suffisantes à elles seules pour alimenter le tissu mou en liquide séreux. La coque peut également être un profilé en pattes d'araignée (figure 10), permettant alors une bonne alimentation en liquide séreux, mais ne contraint pas de manière satisfaisante le volume de croissance du tissu mou. De plus, ce profilé n'offre pas des propriétés mécaniques satisfaisantes.

Le document FINDLAY MICHAEL W. ET AL: "Tissue-Engineered Breast Reconstruction : Bridging the Gap toward Large-Volume Tissue Engineering in Humans",vol. 128, no. 6 1 décembre 2011 (2011-12-01), pages 1206-1215, US (DOI: 10.1097/PRS.0b013e318230c5b2) décrit un dispositif implantable, notamment mammaire, comprenant une coque principale et une chambre de traitement d'au moins un tissu mou, ledit dispositif est en outre configuré pour recevoir au moins un pédiculaire vasculaire, la coque principale comprend un ensemble d'ouvertures traversantes. Ladite coque est faite de polycarbonate et comprend une éponge en PLGA.

Cette technique a pour avantage qu'en une seule intervention chirurgicale, le volume de la poitrine est reconstruit. Le développement du tissu mou s'effectue progressivement, concomitamment à la résorption de la coque résorbable. De plus, le tissu mou implanté autologue comprend un volume de graisse, et non un muscle, tel que par exemple le muscle du grand dorsal. Le prélèvement du tissu mou est moins invasif pour le patient, et les suites chirurgicales sont donc moins lourdes.

Il existe néanmoins un besoin pour améliorer la croissance du tissu mou logé dans la coque, notamment son alimentation, et ce de manière homogène, en liquide séreux riche en facteurs de croissance.

Il existe également un besoin pour un dispositif implantable, notamment mammaire, se résorbant de manière fiable, et totalement au terme d'une période convenue.

Il existe en outre un besoin pour diminuer la quantité de matériaux prothétiques implantés, tout en offrant un dispositif implantable suffisamment résistant mécaniquement, et assurant ses fonctions primaires de croissance du tissu mou, et ce de façon homogène.

Enfin, il existe un besoin pour un dispositif implantable améliorant le confort du patient.

### Exposé de l'invention

La présente invention a pour objet, selon un premier aspect, un dispositif implantable comprenant une coque principale et une chambre de traitement d'au moins un tissu mou, ledit dispositif est outre configuré pour recevoir au moins un pédiculaire vasculaire, la coque principale comprend un ensemble d'ouvertures traversantes et est biorésorbable. Ladite coque principale comprend au moins un (co)polymère biorésorbable ayant un allongement à rupture supérieur ou égal à 200%, comme défini dans les revendications.

La combinaison d'un (co)polymère biorésorbable très souple, ayant un comportement élastique, avec une coque principale délimitant en partie, ou totalement, une chambre de traitement et comprenant un ensemble d'ouvertures traversantes, permet de former une coque principale très résistante en compression, et ce de manière durable.

Avantageusement, la coque principale résiste à un test de fatigue en compression comprenant 325 000 cycles, chaque cycle correspondant à une force en compression de 50 newtons, appliquée à une fréquence de 3,3 Hz. Ce nombre de cycles est estimé comme correspondant à un maintien des propriétés mécaniques sur une durée de 6 mois. Ce nombre de cycles est extrapolé à partir d'une méthode de test de fatigue en compression des implants mammaires comprenant l'application de 6 millions de cycles estimés comme correspondant à un maintien des propriétés mécaniques sur une période de 10 ans.

De préférence, la chambre de traitement est une chambre d'ingénierie tissulaire.

De préférence, la coque principale a une forme sensiblement de dôme.

Dans un mode de réalisation, une première pression manuelle appliquée sur le sommet de la coque principale, en particulier vers la base de la coque principale, entraîne une déformation de cette dernière, ladite coque principale récupère ensuite sa forme initiale, libre de toute contrainte ou sous l'effet d'une seconde pression manuelle appliquée de manière opposée à ladite première pression manuelle.

Avantageusement, la coque principale est suffisamment souple pour pouvoir être déformée ce qui facilite son insertion sur le site à traiter, et permet de réduire la taille de l'incision effectuée, par exemple à environ 4 cm à 5 cm, sur le patient pour son insertion.

Dans un mode de réalisation, le (co)polymère biorésorbable a un allongement à rupture (%) supérieur ou égal à 300%, de préférence supérieur ou égal à 400%, encore de préférence supérieur ou égal à 500%, préférentiellement supérieur ou égal à 700%, en particulier supérieur ou égal à 900%, notamment mesuré selon le sens d'impression, X ou Y, en trois dimensions de l'éprouvette testée.

Dans un mode de réalisation, le (co)polymère biorésorbable a un allongement à rupture (%) inférieur ou égal à 2000%, de préférence inférieure ou égal à 1500%, encore de préférence inférieur ou égal à 1200%, notamment mesuré selon le sens d'impression, X ou Y, en trois dimensions de l'éprouvette testée.

Dans un mode de réalisation, le (co)polymère biorésorbable a un module d'Young supérieur ou égal à 100 MPa, en particulier supérieur ou égal à 150 MPa (de préférence quel que soit le sens d'impression en trois dimensions de l'éprouvette testée : X, Y,Z).

Dans un mode de réalisation, le (co)polymère biorésorbable a un module d'Young inférieur ou égal à 600 MPa, en particulier inférieur ou égal à 400 MPa (de préférence quel que soit le sens d'impression en trois dimensions de l'éprouvette testée : X, Y,Z).

Le module d'Young et l'allongement à rupture sont mesurés selon la norme ASTM D638-14 (« Standard Test Method for Tensile Properties of Plastics ») à une vitesse de traction de 10 mm/min sur des éprouvettes de type V, de préférence à une température de 20°C. Les éprouvettes testées sont de préférence fabriquées par un procédé de fabrication additive, en particulier le sens d'impression peut varier selon les axes X, Y ou Z.

De préférence, la coque principale comprend une surface externe et une surface interne orientée en regard de la chambre de traitement, en particulier la surface interne est sensiblement opposée à la surface externe.

De préférence, les ouvertures traversantes débouchent sur les surfaces externe et interne de la coque principale et s'étendent entre lesdites surfaces externe et interne.

De préférence, les ouvertures traversantes débouchent dans le volume de la chambre de traitement et à l'extérieure de la coque principale, en particulier elles sont agencées dans sa paroi principale, notamment ayant une forme de dôme.

Dans un mode de réalisation, les ouvertures traversantes, désignées dans le présent texte, se présentent sous une seule et même forme ou ont plusieurs formes différentes combinées, en particulier la ou lesdites formes peut/peuvent être choisie(s) parmi : une forme ronde, une forme parallélépipédique tel qu'un carré ou un rectangle, une forme en triangle, une forme de cellule aléatoire, de préférence il s'agit d'une forme sensiblement ronde.

Dans un mode de réalisation, les ouvertures traversantes, notamment sensiblement circulaires, ont au moins une dimension, notamment un diamètre, supérieure ou égale à 1 mm, en particulier inférieure ou égale à 15 mm, plus particulièrement inférieure ou égale à 10 mm, notamment comprise entre 3 mm et 6 mm.

Dans un mode de réalisation, les ouvertures traversantes comprennent des ouvertures traversantes dont la distance séparant des ouvertures traversantes sensiblement circulaires et adjacentes, alignées selon un axe A1, est comprise entre 4 mm et 8 mm ou entre 5 mm et 7 mm, et la distance séparant des ouvertures traversantes sensiblement circulaires et adjacentes, alignées selon un axe A2 sécant avec l'axe A1 en formant un angle d'environ 45°, est comprise entre 6 mm et 10 mm ou entre 7 mm et 9 mm.

De préférence, la coque principale a une paroi principale ayant une épaisseur ecp supérieure à 0 mm, et inférieure ou égale à 8 mm, encore de préférence inférieure ou égale à 5 mm, notamment inférieure ou égale à 3 mm.

De préférence, l'épaisseur de la paroi principale de la coque principale est sensiblement constante.

De préférence, la coque principale a une forme sensiblement hémisphérique, tel un dôme, et comprend une base ayant un diamètre déterminé et une hauteur déterminée (correspondant également à la profondeur de la coque principale).

Dans un mode de réalisation, la base de la coque principale a un diamètre supérieur ou égal à 7 cm et inférieur ou égal à 16 cm, en particulier supérieur ou égal à 9 cm et inférieur ou égal à 14 cm, notamment compris entre 100 mm et 130 mm (bornes supérieure et inférieure comprises).

Dans un mode de réalisation, la hauteur de la coque principale est supérieure ou égale à 1cm, notamment supérieure ou égal à 3 cm, et inférieure ou égale à 6 cm.

La coque principale existe en différentes tailles, et est choisie en fonction des dimensions de la reconstruction finale visée.

Dans un mode de réalisation, la coque principale a une masse supérieure ou égale à 10 g, de préférence inférieure ou égale à 100g, encore de préférence inférieure ou égal à 50 g.

La masse (g) est de préférence déterminée dans le présent texte à une température supérieure ou égale à 20°C et inférieure ou égale à 25°C; en particulier à la pression atmosphérique, par exemple d'1 atm, et notamment avec une humidité relative de 50%.

Dans un mode de réalisation, la coque principale a un volume supérieur ou égal à 150 cm³, de préférence inférieure ou égale à 500 cm³.

On comprend par volume de la coque principale (ou de la coque intermédiaire décrite ci-après), son volume intérieur, délimité par la surface interne, de la coque principal ou intermédiaire.

Avantageusement, on comprend dans le cadre de la présente invention que le volume intérieur de la coque principale, ou le volume de la chambre de traitement, est vide. Cette disposition permet de disposer un ou plusieurs pédicule(s) vasculaire(s) et/ou un ou des matériau(x) (par exemple une ou plusieurs couche(s) textile(s) ou imprimée(s) en 3D) dans ledit volume intérieur de la coque principale ou dans ledit volume de la chambre de traitement.

Par définition, la coque principale et/ou la coque intermédiaire est/sont creuse(s), en particulier la coque principale et/ou la coque intermédiaire comprend/comprennent un volume intérieur ou chambre de traitement permettant de recevoir un ou plusieurs pédicule(s) vasculaire(s) et/ou une ou plusieurs couche(s) de graisse et/ou une ou plusieurs couche(s) poreuses (notamment synthétique(s)), en particulier textile(s) ou imprimée(s) en 3D.

Dans un mode de réalisation, le volume de la chambre de traitement est sensiblement égal au volume intérieur délimité par la coque principale et éventuellement le fond principal, et éventuellement additionné du volume intérieur délimité par la coque intermédiaire.

Dans un mode de réalisation, la coque principale a une force de réaction à la compression statique supérieure ou égale à 400 N (en particulier pour une coque principale dont le volume est supérieur ou égal à 175 cm³ et inférieur ou égal à 475 cm³), en particulier supérieure ou égale à 800 N pour une coque principale dont le volume est supérieur ou égal à 475 cm³.

Dans un mode de réalisation, la coque principale ne présente pas de rupture après un choc de 4,4 Kg répété au moins 3 fois.

Dans un mode de réalisation, la coque principale ne présente pas de rupture après 325 000 cycles de fatigue en compression dans un milieu humide à 37°C, chaque cycle comprenant l'application d'une force de 50 Newtons à 3,3 Hz. Cette valeur est en particulier valable pour une coque principale dont le volume est compris entre 150 cm³ et 500 cm³.

La force de réaction à la compression statique (N), le comportement au choc, et la tenue à la fatigue en compression sont de préférence mesurés selon la norme ISO 14607 :2018 (intitulée : Implants chirurgicaux non actifs - Implants mammaires - Exigences particulières).

Une coque principale témoin a été réalisée dans un polymère d'acide lactique et d'acide glycolique (PLGA (en masse) : 85:15) avec un allongement à rupture nettement inférieur à 200%, en particulier de l'ordre de 5 à 10%. Ladite coque principale témoin, dont le volume est de l'ordre de 200 cm³ ou 300 cm³, se fissure, voire casse au test au choc et au test en fatigue en compression précité entre 20 000 et 50 000 cycles.

La coque principale selon l'invention, malgré sa forme, son faible poids, et les nombreuses ouvertures traversantes, présente de bonnes performances mécaniques obtenues en utilisant un (co)polymère très souple et biorésorbable.

Ledit (co)polymère biorésorbable peut être un copolymère ou un terpolymère.

De préférence, ledit (co)polymère comprend au moins deux unités de répétition différentes.

De préférence, ledit (co)polymère est un polymère de ε-caprolactone et d'au moins une unité de répétition différente du ε-caprolactone, par exemple issue de l'acide lactique et/ou de l'acide glycolique.

De préférence, les unités de répétition issues de l'acide lactique peuvent être des unités de répétition d'acide lactique de forme L et/ou des unités de répétition d'acide lactique de forme D et/ou de forme D,L.

Ledit au moins un (co)polymère comprend des unités de répétition du ε-caprolactone dont la fraction molaire (dans ledit (co)polymère) est inférieure ou égale à 50%, encore de préférence inférieure ou égale à 40%, en particulier entre 20% et 40% (bornes comprises).

De préférence, ledit au moins un (co)polymère comprend des unités de répétition d'acide lactique de forme L et/ou des unités de répétition d'acide lactique de forme D et/ou de forme D,L dont la fraction molaire (dans ledit (co)polymère) est supérieure ou égale à 50%, encore de préférence supérieure ou égale à 60%, en particulier entre 60% et 80% (bornes comprises).

Lesdites fractions molaires peuvent être déterminées par spectroscopie RMN.

De préférence, ledit au moins un (co)polymère comprend des unités de répétition, d'acide lactique de forme L, et/ou, des unités de répétition d'acide lactique de forme D et/ou de forme D,L, dont la fraction molaire est inférieure ou égale à 90%, encore de préférence inférieure ou égale à 80%.

Dans un mode de réalisation, la coque intermédiaire et/ou le fond principal décrit(s) ci-après comprend/comprennent (chacun) ledit au moins un (co)polymère biorésorbable.

De préférence, la fraction massique dudit au moins un (co)polymère biorésorbable dans la coque principale (c'est-à-dire par rapport à la masse totale de la coque principale), et/ou dans le fond principal (c'est-à-dire par rapport à la masse totale du fond principal), et/ou dans la coque intermédiaire (c'est-à-dire par rapport à la masse totale de la coque intermédiaire), est supérieure ou égale à 50%, encore de préférence supérieure ou égale à 80%, préférentiellement supérieure ou égale à 90%, notamment de l'ordre de 100%.

Dans un mode de réalisation, la coque principale, et éventuellement le fond principal et/ou la coque intermédiaire, est(sont) essentiellement constituée(s) dudit au moins un (co)polymère résorbable.

De préférence, la coque principale délimite un espace interne formant au moins une partie, ou sensiblement la totalité, de la chambre de traitement. La coque principale protège ainsi la croissance du tissu mou. Le volume de l'espace interne délimité au moins partiellement par la coque principale est comblé au fur et à mesure de la croissance du tissu mou. La croissance et donc l'expansion du tissu sont donc contrôlées par le volume de la chambre de traitement. La chambre de traitement permet ainsi la croissance de cellules aptes à former un tissu mou, ce nouveau tissu mou est formé in situ dans l'organisme vivant dans lequel le dispositif est implanté afin de remplacer et/ou augmenter un volume de tissu du corps du sujet.

Le tissu mou comprend au moins une couche de cellules choisies par les adipocytes, les cellules capables de se différencier en adipocytes, et les mélanges de ces deux types de cellules.

On comprend dans le présent texte par « tissu mou », les tissus adipeux, et plus particulièrement le tissu adipeux profond, par exemple prélevé par liposuccion.

Les cellules précitées sont de préférence obtenues par utilisation du surnageant après centrifugation d'un amas graisseux prélevé sur le sujet lui-même. Il s'agit d'une greffe autologue limitant les réactions de rejet.

On comprend par « cellules capables de se différencier en cellules adipeuses » dans le présent texte, les cellules souches adultes, notamment les cellules souches mésenchymateuses adultes (i.e provenant d'un sujet adulte) qui sont aptes à se différencier en cellules pouvant se différencier en adipocytes.

Il est possible également que le dispositif implantable comprenne au moins une couche poreuse combinée avec au moins une couche de cellules choisies parmi les adipocytes. Ladite couche poreuse peut être un ensemble d'au moins deux couches empilées, lesdites couches étant obtenues par l'enchevêtrement de fils et/ou de fibres lié(e)s selon des points de liaison. Ladite au moins une couche de cellules choisies par les adipocytes est de préférence une couche intermédiaire disposée entre lesdites au moins deux couches poreuses. Ladite au moins une couche poreuse peut être choisie parmi les textiles (tricot, tissu, nontissé), par exemple être éléctrofilée, ou être imprimée en trois dimensions, ou une combinaison de ces derniers.

Ladite au moins une couche poreuse combinée avec au moins une couche de cellules choisies parmi les adipocytes est de préférence disposée dans la chambre de traitement, notamment dans la chambre de traitement supérieure et/ou dans la chambre de traitement inférieure (cf. ci-après).

On comprend dans le présent texte, par « points de liaison », l'ensemble des points de contact entre fils et/ou fibres, ces points de contact étant obtenus mécaniquement (i.e par tricotage, tissage, aiguilletage,...) ou chimiquement (par fusion par exemple du ou des fils et/ou fibres entre-eux(elles) et/ou par collage, à l'aide par exemple d'un liant).

De préférence, la coque principale, et encore de préférence, le fond principal et/ou la cloison supérieure de réception et/ou la coque intermédiaire et/ou la cloison latérale, et/ou le ou les fils et/ou les fibres, est/sont chacun, dans un ou plusieurs matériau(x) biorésorbable(s).

Le ou les matériau(x) bio-résorbable(s), dont ledit au moins un (co)polymère biorésorbable, est/sont, ainsi que la configuration de la coque principale et/ou du fond principal et/ou de la coque intermédiaire, et des cellules traversantes, de préférence déterminés en sorte d'obtenir une bio-résorption, et notamment du dispositif implantable, au terme de 6 mois, ou par exemple au terme de 12 mois ou 18 mois.

Le ou les matériau(x) biorésorbable(s), en particulier ledit au moins un (co)polymère biorésorbable, est/sont choisi(s) dans la liste comprenant : les polyesters, notamment les polyesters aliphatiques, par exemple le polydioxanone, ou en particulier les polymères issus de la (co)polymérisation d'au moins un monomère choisi par : un monomère d'acide glycolique, un monomère de lactide de forme L, D ou D,L; un monomère de ε-caprolactone ; ou un mélange de ces derniers ; encore de préférence parmi un polymère d'acide lactique de forme L (PLLA) ou D (PLDA) ou D,L (PDLLA) ou un mélange de ces derniers ; un polymère d'acide glycolique (PGA) ; un copolymère d'acide lactique de forme L et/ou D et/ou D,L, et d'acide glycolique ; un copolymère d'ε-caprolactone et de lactide de forme L et /ou D et/ou L,D.

De préférence, ledit au moins un (co)polymère a une température de transition vitreuse inférieure à 50°C, encore de préférence comprise entre 25°C et 40°C, en particulier mesurée à une vitesse de 10°C/min selon la norme ISO 11357-1 : 2016 et USO 11357-2 :2020 (intitulée Plastiques - Analyse calorimétrique différentielle (DSC) - Partie 2 : Détermination de la température et de la hauteur de palier de transition vitreuse).

On comprend dans le présent texte par « résorbable » ou « biorésorbable », tout objet (matériau, coque,...) ayant la propriété de se dégrader lorsqu'il est implanté dans le corps d'un sujet vivant, les produits issus de la dégradation étant évacués par l'organisme du sujet vivant de manière à ce que ledit objet ait été totalement évacué au terme d'une période déterminée (fonction de la nature, de la quantité et de la structure de l'objet notamment), par exemple au terme de l'ordre de 3 mois ou de 6 mois ou de 18 mois.

De préférence, la coque principale, et/ou la coque intermédiaire, comprend/comprennent au moins une ouverture traversante latérale, éventuellement deux ouvertures traversantes latérales, formant au moins un passage transversal permettant l'insertion d'au moins un pédicule vasculaire. Une portion du pédicule vasculaire est ainsi placée dans la chambre de traitement en passant à travers une ouverture traversante latérale, et en partie dans l'espace interne de la coque principale ou intermédiaire, et une autre portion dudit pédicule vasculaire se projette à l'extérieur de la coque principale ou intermédiaire. Le pédicule vascularisé sert ainsi d'élément intermédiaire d'alimentation entre la région du corps recevant le dispositif implantable, et les cellules du tissu mou disposé dans la chambre de traitement.

De préférence, la ou les ouverture(s) traversante(s) latérale(s) est/sont une ou des fenêtre(s) d'accès au volume intérieur de la coque principale, ou de la coque intermédiaire. La ou les ouverture(s) traversante(s) latérale(s) a/ont au moins dimension supérieure, notamment supérieure d'au moins 3 fois ou 5 fois, à l'une des dimensions desdites ouvertures traversantes.

On entend par « surface interne » dans le présent texte, toute surface orientée vers la chambre de traitement. Par opposition, on entend par « surface externe » dans le présent texte, toute surface orientée à l'extérieure de la coque principale ou de la coque intermédiaire ou du fond principal décrite ci-après.

Dans une variante, ledit copolymère biorésorbable est un copolymère de ε-caprolactone et de lactide de forme L, D ou L,D.

La Demanderesse a déterminé que ce polymère permet d'atteindre les performances mécaniques visées dans le cadre de la présente invention, en particulier la tenue à la fatigue, et la résistance au choc.

Ledit copolymère peut s'écrire sous la forme poly (L-lactide ou D-lactide ou L,D-lactide-co-ε-caprolactone), en particulier poly (L-lactide-co-ε-caprolactone).

Dans une variante, ledit (co)polymère biorésorbable a une masse molaire moyenne en nombre Mn supérieure ou égale à 10 000 g/mol, de préférence supérieure ou égale à 25 000 g/mol, encore de préférence supérieure ou égale à 45 000 g/mol, en particulier supérieure ou égale à 65 000 g/mol.

Dans une variante, ledit (co)polymère biorésorbable a une masse molaire moyenne en nombre Mn inférieure ou égale à 200 000 g/mol, de préférence inférieure ou égale à 175 000 g/mol, encore de préférence inférieure ou égale à 150 000 g/mol, en particulier inférieure ou égale à 120 000 g/mol ou 100 00 g/mol.

Dans une variante, ledit (co)polymère biorésorbable a une masse molaire moyenne en masse Mw supérieure ou égale à 50 000 g/mol, de préférence supérieure ou égale à 75 000 g/mol, encore de préférence supérieure ou égale à 100 000 g/mol, en particulier supérieure ou égale à 125 000 g/mol.

Dans une variante, ledit (co)polymère biorésorbable a une masse molaire moyenne en nombre Mw inférieure ou égale à 300 000 g/mol, de préférence inférieure ou égale à 250 000 g/mol, encore de préférence inférieure ou égale à 200 000 g/mol, en particulier inférieure ou égale à 180 000 g/mol ou 100 00 g/mol.

Dans une variante, l'indice de polydispersité dudit au moins un (co)polymère biorésorbale est inférieur ou égal à 2,5 ou 2,3 ou 2 ou 1,8.

Dans une variante, l'indice de polydispersité (I= Mw/Mn) du (co)polymère biorésorbable est supérieur ou égal à 1 ou 1,3 ou 1,5.

Dans une variante, la coque principale a une surface externe, et le rapport de, la superficie totale (mm²) des ouvertures traversantes, sur la superficie totale de la surface externe de la coque principale, est supérieur ou égal à 35%, de préférence inférieur ou égal à 60%.

Avantageusement, la masse de la coque principale est optimisée afin qu'elle satisfasse les performances mécaniques visées tout en permettant de contrôler la durée de résorption, et améliorer le confort du patient.

De plus, cette porosité liée aux cellules traversantes favorise la circulation homogène de liquide séreux riche en facteurs de croissance vers le tissu mou à développer.

La structure de la coque principale, et éventuellement la structure de la coque intermédiaire, apporte(nt) avantageusement les propriétés mécaniques suffisantes pour maintenir un volume une fois implanté sensiblement également au volume de la chambre de traitement, au moins durant la durée de la bio-résorption. Cette disposition permet avantageusement d'obtenir également une reconstruction esthétique immédiate pour le sujet, en une seule intervention chirurgicale.

La coque principale, et éventuellement la coque intermédiaire, doit/doivent cependant comprendre une porosité liée aux ouvertures traversantes supérieure à un certain seuil afin qu'elle ait des propriétés mécaniques suffisantes, et ce pendant toute la résorption éventuelle de la coque principale, pour orienter, et protéger, le développement du tissu mou.

De préférence, la surperficie totale et la superficie ouverte de la coque principale ou de la coque intermédiaire sont estimées par l'intermédiaire du logiciel de conception SOLIDWORKS 2019-SPO4 (la procédure utilisée est :1/onglet « evaluate », 2/ Sélection de la surface, 3/option « mesure »,4/valeur de surface).

De préférence, la superficie totale (mm²) des cellules traversantes débouchant sur la surface externe de la coque principale est sensiblement égale à la superficie totale des cellules traversantes débouchant sur la surface interne de la coque principale. L'épaisseur de la paroi principale délimitant lesdites ouvertures traversantes est donc sensiblement constante.

De préférence, la superficie des surfaces externe et/ou interne de la coque principale, de la coque intermédiaire, des cellules traversantes, de la paroi latérale, et de la paroi supérieure de réception (ci-après), est/sont mesurées en considérant ces pièces à plat, ayant une épaisseur sensiblement égale à 2 mm.

Dans une variante de réalisation, la coque principale a une surface externe, et le rapport de, la superficie de la surface externe pleine de la coque principale, sur la superficie totale de la surface externe de la coque principale, est supérieur ou égal à 40%, de préférence supérieur ou égal à 50%, en particulier inférieur ou égal à 70%.

On comprend par surface externe pleine, la surface occupée par la structure ou le squelette de la coque principale ou intermédiaire.

Dans une variante de réalisation, le squelette de la coque principale a un taux de remplissage supérieur ou égal à 70%, encore de préférence supérieur ou égal à 80%, encore de préférence supérieur ou égal à 90%, notamment de l'ordre de 100%.

Lorsque le taux de remplissage est par exemple de l'ordre de 70%, cela signifie que le taux de vide dans le squelette de la coque principale est de l'ordre de 30%.

Ce taux de remplissage s'applique également de préférence au squelette de la coque intermédiaire et/ou au squelette du fond principal.

Cette disposition permet avantageusement d'améliorer les propriétés mécaniques de la coque principale et/ou de la coque intermédiaire et/ou du fond principal.

De préférence, la coque principale et/ou la coque intermédiaire et/ou le fond principal est/sont fabriqué(e)(s) par un procédé de fabrication additive permettant de réguler le taux de remplissage. L'ensemble des ouvertures traversantes apportent la porosité nécessaire à l'implant. Il n'est pas nécessaire d'augmenter encore la porosité en ayant un taux de remplissage faible ce qui permet d'améliorer les performances mécaniques.

Dans une variante de réalisation, le dispositif comprend un fond principal ayant des ouvertures traversantes et formant une partie postérieure de la chambre de traitement.

De préférence, la coque principale forme une partie antérieure de la chambre de traitement.

Dans un mode de réalisation, le fond principal comprend une face interne (en particulier en regard de la chambre de traitement) et une face externe, opposée à la face interne, lesdites ouvertures traversantes s'étendent entre lesdites faces interne et externe et débouchent sur ces dernières.

Au moins un lambeau de tissu mou est ainsi maintenu dans la chambre de traitement entre d'une part le fond principal et la coque principale. L'accès de graisse et/ou de tissu(s) non souhaité(s) dans la chambre de traitement est ainsi empêché.

De préférence, le fond principal a une masse supérieure à 0 g et inférieure ou égale à 20 g, encore de préférence inférieure ou égale à 12 g.

De préférence, le fond principal est biorésorbable.

Dans une variante de réalisation, le dispositif comprend une coque intermédiaire comprenant des ouvertures traversantes et une cloison supérieure de réception subdivisant la chambre de traitement entre une chambre supérieure de traitement et une chambre inférieure de traitement, ledit dispositif étant configuré pour que chacune desdites chambres de traitement supérieure et inférieure soit apte à recevoir au moins un pédicule vasculaire.

De préférence, la coque intermédiaire est biorésorbable.

Cette disposition permet avantageusement d'augmenter le volume de la chambre de traitement en espaçant le ou les tissu(s) mou(s) disposé(s) dans la chambre de traitement supérieure, de celui ou de ceux disposé(s) dans la chambre de traitement inférieure. Il est ainsi possible de favoriser une reconstruction d'un volume de l'ordre de 500 cm³ à 700-800 cm³. En effet, pour reconstruire un volume de tissu mou ayant un volume important, par exemple de 400 cm³ et plus, les inventeurs ont découvert qu'il est préférable de subdiviser ce volume, afin de former des chambres de traitement supérieure et inférieure, chacune recevant de préférence un ensemble de couche(s) poreuse(s) avec un ensemble de couche(s) de cellules choisies parmi les adipocytes, ledit ensemble étant en liaison et alimenté par au moins un pédicule vasculaire.

Dans un mode de réalisation, la coque intermédiaire a une forme tronconique.

Dans un mode de réalisation, la cloison supérieure de réception comprend une face interne (en particulier orientée en regard de la chambre de traitement) et une face externe, sensiblement opposée à la face interne, et lesdites ouvertures traversantes de la coque intermédiaire s'étendent entre les faces interne et externe de la cloison supérieure de réception et débouchent sur ces dernières.

Dans variante de réalisation, ladite cloison supérieure de réception est espacée du fond principal d'une distance d supérieure à 0 mm afin de délimiter au moins en partie la chambre de traitement inférieure.

Dans une variante de réalisation, la coque intermédiaire comprend une cloison latérale se projetant d'une face inférieure de la paroi supérieure de réception, en particulier dans la chambre de traitement inférieure, plus particulièrement se projetant vers le fond principal.

De préférence, la cloison latérale comprend une surface interne et une surface externe, et des ouvertures traversantes s'étendant entre lesdites surfaces interne et externe, et débouchant sur ces dernières.

De préférence, le rapport de la superficie totale (mm²) des ouvertures traversantes débouchant sur la surface externe de la cloison latérale, sur la superficie de la surface externe de la cloison latérale est supérieur ou égal à 40%, de préférence inférieur ou égal à 60%, encore de préférence inférieur ou égal à 50%.

La paroi principale de la cloison latérale a de préférence une épaisseur ecl supérieure à 0 mm, et inférieure ou égale à 5 mm, encore de préférence inférieure ou égale à 3 mm.

La superficie totale (mm²) (et donc la taille) des ouvertures traversantes débouchant sur la surface externe de la cloison latérale est sensiblement égale à la superficie totale des ouvertures traversantes débouchant sur la surface interne du rebord périphérique. L'épaisseur de la paroi principale délimitant lesdites ouvertures traversantes est donc sensiblement constante.

De préférence, le squelette de la coque intermédiaire a un taux de remplissage supérieur ou égal à 70%, encore de préférence supérieure ou égale à 80%, encore de préférence inférieure ou égale à 90%.

Dans une variante, le fond principal comprend un ou plusieurs logements, ouvert(s) vers la chambre de traitement, et configuré(s) pour recevoir, notamment par emboîtement, une partie ou des parties d'un bord inférieur, notamment d'un bord annulaire inférieur, de la coque principale ou de la coque intermédiaire.

Cette disposition facilite la solidarisation du fond principal avec la coque principal ou intermédiaire.

Dans une variante, le fond principal comprend :
- un rebord externe , continu ou discontinu, se projetant d'une face interne du fond principal dans la chambre de traitement, en particulier dans la chambre de traitement inférieure, en particulier selon le bord périphérique du fond principal, et
- une ou plusieurs portions de paroi vertical se projetant de la face interne du fond principal dans la chambre de traitement, et délimitant au moins en partie avec ledit rebord externe, le ou lesdits logement(s).

Dans une variante, la coque principale ou la coque intermédiaire comprend un bord externe inférieur et un bord interne inférieur en retrait dudit bord externe inférieur. De préférence, le bord interne inférieur est configuré pour coopérer, par emboitement, avec le ou lesdits logement(s) de réception du fond principal.

Dans une variante, le bord externe inférieur vient en appui sur le rebord externe du fond principal.

Dans une variante, la coque principale et/ou la coque intermédiaire comprend/comprennent chacun une ou des ouvertures de solidarisation, notamment adjacentes au bord inférieur externe, et le fond principal comprend une ou des ouvertures de solidarisation, notamment adjacentes au bord périphérique dudit fond principal, lesdites ouvertures de solidarisation étant configurées en sorte de permettre le passage d'au moins un organe de solidarisation, tel que un fil de suture, à travers une ouverture de solidarisation de la coque principale et une ouverture de solidarisation du fond principal.

De préférence, lesdites ouvertures de solidarisation sont des ouvertures traversantes, telles que définies dans le présent texte.

Les différents ratios indiqués dans le présent texte en regard des ouvertures traversantes comprennent toutes les ouvertures traversant la coque principale ou intermédiaire ou le fond principal en s'étendant entre les surfaces externe et interne. Dans une variante de réalisation, au moins une partie des ouvertures traversantes de la coque principale et/ou de la coque intermédiaire et/ou du fond principal sont des polyèdres de Voronoï.

Les ouvertures traversantes sont avantageusement distribuées selon la surface externe (et donc sur la surface interne) de la coque principale, et éventuellement de la surface externe et de la surface interne de la coque intermédiaire, selon le diagramme de Voronoï.

Les inventeurs ont découvert que cette disposition permet d'optimiser la surface totale des ouvertures traversantes, ainsi que les propriétés mécaniques de la coque principale, et/ou de la coque intermédiaire.

En effet, la répartition et la taille desdites ouvertures traversantes sont déterminées de manière aléatoire, les contraintes exercées vont alors se distribuer également de manière aléatoire sur l'implant lors sa sollicitation.

De préférence, la longueur, la largeur et le nombre des cellules de Voronoi sont définies dans le langage de programmation Python comprenant une librairie dédiée aux cellules de Voronoi, puis le profil obtenu desdites cellules est projeté sur la coque principale ou la coque intermédiaire pour définir les ouvertures traversantes selon l'invention.

Dans une variante de réalisation, la coque principale comprend au moins deux arceaux verticaux, en particulier s'étendant à partir d'un sommet de la coque principale, et des sections transversales, notamment au moins partiellement annulaires, s'étendant entre lesdits arceaux verticaux et en liaison avec ces derniers.

Les extrémités distales libres des arceaux verticaux sont de préférence reliées par une section transversale de base, au moins partiellement annulaire.

De préférence, la section transversale de base est dans un plan d'appui.

De préférence, les sections transversales sont arrangées selon des ensembles de sections transversales, les sections transversales d'un ensemble étant disposées sensiblement dans un plan transversal, lesdits ensembles étant disposés dans des plans transversaux sensiblement parallèles entre-eux, et notamment sensiblement parallèles au plan d'appui.

Cette disposition peut se retrouver également dans la coque intermédiaire. Les arceaux verticaux s'étendent dans ce cas de la périphérie de la paroi supérieure de réception.

Dans une variante de réalisation, le fond principal et/ou la cloison supérieure de réception, notamment sensiblement plane(s), comprend/ comprennent chacun, des ouvertures traversantes agencées pour définir plusieurs premiers motifs liés les uns aux autres par des premières structures de liaison.

Dans une variante de réalisation, les premiers motifs comprennent un ou plusieurs segment(s), notamment au moins trois segments et au plus six segments, au moins l'un du ou des segment(s) de chaque premier motif est solidarisé à une première structure de liaison.

De préférence, lesdits segments sont sensiblement parallèles entre-eux, et espacés les uns des autres.

De préférence, le ou les segment(s) a/ont (chacun) une largeur inférieure ou égale à 3 mm, en particulier inférieure ou égale à 2 mm, plus particulièrement supérieure ou égale à 0,10 mm, notamment supérieure ou égale à 0,30 mm.

De préférence, le ou les segment(s) a/ont (chacun) une longueur inférieure ou égale à 20 mm, en particulier inférieure ou égale à 15 mm, plus particulièrement supérieure ou égale à 3 mm, notamment supérieure ou égale à 6 mm.

De préférence, la distance séparant deux segments adjacents d'un premier motif est supérieure ou égale à 0,1mm et inférieure ou égale à 2 mm.

Dans une variante de réalisation, les premières structures de liaison sont chacune reliées à au moins trois premiers motifs distincts.

Dans une variante de réalisation, le fond principal et/ou la cloison supérieure de réception comprend/comprennent chacun, des secondes structures de liaison, différentes des premières structures de liaison, chacune des secondes structures de liaison est reliée à au moins deux segments d'un même premier motif.

Dans une variante de réalisation, au moins l'une des secondes structures de liaison est/sont reliée(s) à au moins trois premiers motifs distincts. De préférence, l'ensemble des secondes structures de liaison sont reliées à au moins trois premiers motifs distincts.

Dans une variante de réalisation, la coque principale et/ou la coque intermédiaire comprend/comprennent chacune une ou plusieurs ouverture(s) traversante(s) et transversale(s) débouchant dans la chambre de traitement, et permettant le placement d'au moins un pédicule vasculaire.

Ces ouvertures transversales sont de préférence considérées comme des ouvertures traversantes, telles que définies dans le présent, pour le calcul de la porosité.

Dans une variante de réalisation, la coque principale a une forme sensiblement de dôme.

Dans une variante, l'un au moins de ladite coque principale, de ladite coque intermédiaire, dudit fond principal, et de ladite cloison supérieure de réception, ou une combinaison de ces derniers, est obtenu(e) par fabrication additive ou soustractive, de préférence additive.

On comprend par une étape de fabrication additive, toute fabrication par ajout ou agglomération d'un ou plusieurs matériau(x), notamment biorésorbable(s), et ce par addition de couches successives de matériau(x) sous contrôle d'un organe d'application contrôlé par un programme d'ordinateur mis en œuvre par un ordinateur (par exemple frittage sélectif par laser ou Selective Laser Sintering, c'est-à-dire une poudre fusionnée ou fritée grâce à l'énergie d'un laser de forte puissance, comme un laser CO₂).

De préférence, la ou les squelette(s) de la coque principale et/ou de la coque intermédiaire et/ou du fond principal, et/ou de la paroi supérieure de réception et/ou de la paroi latérale a/ont chacun un taux de remplissage supérieur ou égal à 70%, encore de préférence supérieur ou égal à 80%, encore de préférence supérieur ou égal à 90%, notamment de l'ordre de 100%.

La présente invention a pour objet, selon un second aspect, un procédé de fabrication d'un dispositif implantable, en particulier selon l'une quelconque des variantes de réalisation en référence au premier aspect de l'invention, ledit procédé comprend une étape de fabrication additive ou soustractive, de préférence additive, de ladite coque principale, et éventuellement de ladite coque intermédiaire et/ou du fond principal, avec au moins un fil comprenant ledit (co)polymère ayant un allongement à la rupture supérieure ou égale à 200%, comme défini dans les revendications.

De préférence, la fraction massique dudit au moins un (co)polymère biorésorbable tel que défini dans ledit au moins un fil est supérieure ou égale à 80% ou 90%, notamment de l'ordre de 100%.

La présente invention a également pour objet, selon un troisième aspect, un procédé de fabrication ex-vivo d'un dispositif implantable pour le remplacement et/ou pour l'augmentation d'un volume de tissu mou comprenant :
- la fourniture d'un dispositif implantable selon l'une quelconque des variantes de réalisation en référence au premier aspect de l'invention, et
- la disposition dans la chambre de traitement de cellules choisies parmi les adipocytes, les cellules aptes à se différencier en adipocytes et les mélanges de ces deux types de cellules, lesdites cellules provenant de préférence dudit sujet.

Les variantes de réalisation, ainsi que les définitions et modes de réalisation selon un premier aspect de l'invention peuvent être combinées entre-eux indépendamment les uns des autres, et avec les variantes selon un second aspect et/ou un troisième aspect de l'invention.

### Description des dessins

L'invention sera mieux comprise à la lecture de la description qui suit d'exemples de réalisation de l'invention donné à titre d'exemples non limitatifs, en référence aux dessins annexés, sur lesquels :
- la figure 1 représente de manière schématique, vue en perspective, la coque principale d'un premier exemple de dispositif implantable selon l'invention;
- la figure 2 représente de manière schématique, vue en perspective, le fond principal du premier exemple de dispositif implantable de la figure 1;

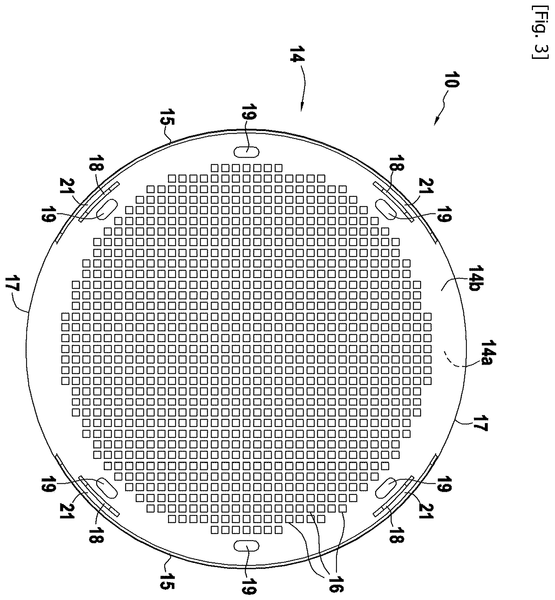

- la figure 3 représente de manière schématique, vue de dessus, le fond principal du premier exemple de dispositif implantable des figures 1 et 2;
- la figure 4 représente de manière schématique, en perspective, un second exemple de dispositif implantable selon l'invention ;
- la figure 5 représente de manière schématique, en perspective, la coque principale du second exemple de dispositif implantable représenté à la figure 4 ;
- la figure 6 représente schématiquement, vue de dessus, le fond principal du second exemple de dispositif implantable représenté sur les figures 4 et 5 ;
- la figure 7 illustre de manière schématique un agrandissement des motifs géométriques du fond principal représenté à la figure 6;
- la figure 8 représente de manière schématique une variante de la coque principale du premier ou second exemple de dispositif implantable selon l'invention;
- la figure 9 représente de manière schématique une variante du fond principal du premier ou second exemple de dispositif implantable selon l'invention.

### Description des modes de réalisation

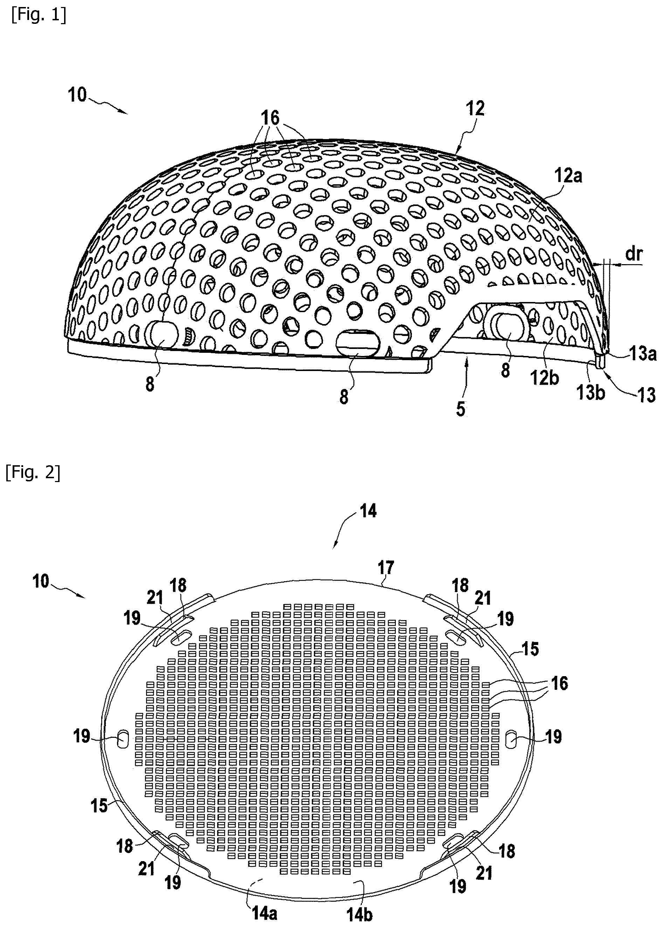

Le premier exemple de dispositif implantable 10 selon l'invention comprend une coque principale 12 représentée à la figure 1 et un fond principal 14 chacun pourvu d'ouvertures traversantes 16. La coque principale 12 lorsqu'elle coopère avec le fond principal 14 délimite une chambre de traitement 5 configurée pour recevoir au moins un tissu mou. Le fond principal 14 forme une partie postérieure du dispositif implantable 10 et la coque principale 12 forme une partie antérieure du dispositif implantable 10. La coque principale 12 et le fond principal 14 sont biorésorbables, et comprennent chacun au moins un (co)polymère biorésorbable ayant un allongement à rupture supérieur ou égal à 200%, dans cet exemple précis de l'ordre de 900-1000% dans les sens X et Y de l'éprouvette testée, notamment de type V, selon la norme ASTM D638.14 à une vitesse de traction de 10 mm/min. Le module d'Young dudit polymère est de préférence supérieur ou égal à 150 MPa, que ce soit dans les sens X, Y et Z de l'éprouvette, notamment de type V selon la norme ASTM D638.14 à une vitesse de traction de 10 mm/min.

Dans cet exemple précis, la coque principal 12 et le fond principal 14 sont chacun obtenus par une méthode de fabrication additive, par la mise en œuvre d'un monofilament dans ledit polymère souple, en particulier dans un copolymère de poly(L-lactide-co-ε-caprolactone) dont la fraction molaire en ε-caprolactone est comprise entre 25% et 35%.

La coque principale 12 et le fond principal 14 comprennent des ouvertures traversantes 16 afin de favoriser les échanges en liquide séreux et autres facteurs de croissance entre l'environnement du dispositif 10 une fois implanté et la chambre de traitement 5.

La coque principale 12 a une surface externe 12a et une surface interne 12b, le rapport de, la superficie totale (mm²) des ouvertures traversantes 16, sur la superficie totale (mm²) de la surface externe 12a de la coque principale 12, est supérieur ou égal à 35% et inférieur ou égal à 60%, en particulier compris entre 40% et 45%. Ce rapport est équivalent à un taux d'ouverture.

Le rapport de, la superficie de la surface externe 12a pleine de la coque principale 12 , sur la superficie totale de la surface externe 12a de la coque principale 12, est supérieur ou égal à 40% et inférieur ou égal à 70%, en particulier compris entre 55% et 60%. Ce rapport est équivalent à un taux de fermeture.

Le même taux d'ouverture que celui précité s'applique au fond principal 14 pour sa face externe 14a ou sa face interne 14b, c'est-à-dire de l'ordre de 40% à 45%. Le même taux de fermeture que celui précité s'applique au fond principal 14 pour sa face externe 14a ou interne 14b, c'est-à-dire de l'ordre de 55% à 60%.

La coque principale 12 et le fond principal 14 ont un squelette (ou structure pleine) dont le taux de remplissage est de l'ordre de 100%.

Le fond principal 14 comprend des logements 21 ouverts vers la chambre de traitement 5, et configurés pour recevoir par emboîtement des parties du bord inférieur 13, en particulier du bord annulaire inférieure 13, de la coque principale 12. En particulier, le fond principal 14 comprend un rebord externe 15, discontinue dans cet exemple précis, se projetant de la face interne 14b du fond principal 14 dans la chambre de traitement 5 selon le bord périphérique 17 du fond principal 14. Le fond principal 14 comprend également les portions de paroi vertical 18 se projetant de la face interne 14b du fond principal 14 dans la chambre de traitement 5, et délimitant au moins en partie avec ledit rebord externe 15, lesdits logements 21.

De préférence, la coque principale 12 comprend un bord externe inférieur 13a et un bord interne inférieur 13b en retrait dudit bord externe inférieur 13a, notamment d'une distance dr, par exemple de l'ordre de 1mm ou 2 mm. Le bord interne inférieur 13b est configuré pour coopérer, par emboitement, avec les logements de réception 21. Le bord externe inférieur 13a vient en appui sur le rebord externe 15 du fond principal 14. Le bord inférieur 13 de la coque principale 12 coopère ainsi avec le fond principal 14 pour leur liaison. Le bord inférieur externe 13a de la coque principale 12 vient en appui sur la face supérieure du rebord externe 15, et présente de préférence une largeur dr de l'ordre de celle du rebord externe 15 de sorte que la surface externe 12a de la coque principale 12 se prolonge par la face externe du rebord externe 15 sans créer de protrusion à leur jonction. Cet agencement permet de positionner parfaitement la coque principale 12 par rapport au fond principal 14.

Enfin, la coque principale 12 comprend des ouvertures traversantes de solidarisation 8, dans cet exemple au nombre de six. Ces ouvertures de solidarisation 8 sont adjacentes au bord inférieur 13, en particulier au bord inférieur externe 13a. Le fond principal 14 comprend également des ouvertures de solidarisation 19, dans cet exemple au nombre de six, lesquelles sont adjacentes au bord périphérique 17. Lesdites ouvertures de solidarisation 8 et 19, qui sont traversantes, sont configurées en sorte de permettre le passage d'au moins un organe de solidarisation (non représenté), tel qu'un fil de suture, à travers une ouverture de solidarisation 8 de la coque principal 12 et une ouverture de solidarisation 19 du fond principal 14. Dans cet exemple, six organes de solidarisation, par exemple agrafes ou fils de suture, sont disposés par paire d'ouvertures comprenant une ouverture de solidarisation 8 et une ouverture de solidarisation 19. La coque principale 12 et le fond principal 14 sont ainsi solidarisés ensemble solidement une fois ledit au moins un tissu mou et d'autres éléments placés dans la chambre de traitement 5. Le premier dispositif implantable est optimisé en termes de masse, de taux d'ouvertures, et de performances mécaniques (résistance au choc, tenue à la fatigue,..) ce qui permet d'offrir un dispositif fiable, une croissance dudit au moins un tissu mou homogène, et contrôlée, et d'améliorer le confort du patient.

Par exemple pour un volume d'une chambre de traitement 5 de 475 cm³, la masse de la coque principale 12 est de 38 g et la masse du fond principal 14 est de 9 g.

Par exemple pour un volume d'une chambre de traitement 5 de 175 cm³, la masse de la coque principale 12 est de 19 g et la masse du fond principal 14 est de 6 g.

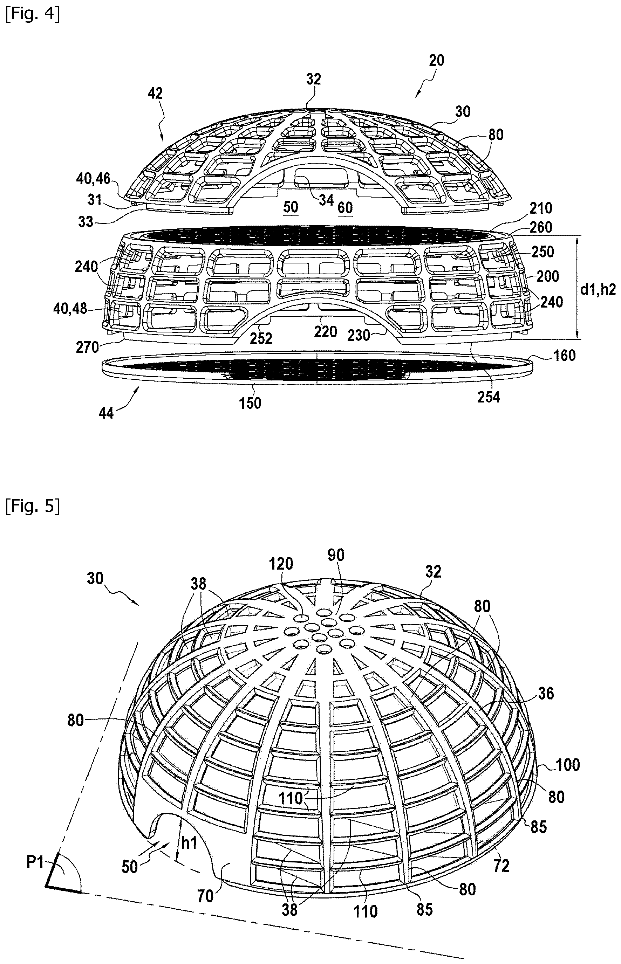

Le second exemple de dispositif implantable 20 représenté à la figure 4 permet la formation d'une chambre de traitement ayant un volume supérieur au volume de la chambre de traitement du premier exemple de dispositif 10.

Avantageusement, le dispositif 10 comprend deux ouvertures traversantes latérales, dont l'une est visible sur la figure 1, pour permettre le placement de deux pédicules vasculaires, un pédicule vasculaire étant passé en partie à travers une ouverture traversante latérale.

Ce dispositif 20 comprend une coque principale 30 et une chambre de traitement d'un tissu mou 40. Ledit dispositif 20 est outre configuré pour recevoir au moins un pédiculaire vasculaire à l'aide d'une première ouverture transversale 50 et d'une seconde ouverture transversale 60, chacune débouchant dans la chambre de traitement 40 et à l'extérieur de la coque principale 30. La coque principale 30 a une surface externe 32 et une surface interne 34 et comprend un ensemble d'ouvertures traversantes 38 s'étendant entre lesdites surfaces interne 34 et externe 32 et débouchant sur ces dernières. De préférence, le rapport de, la superficie totale (mm²) des ouvertures traversantes 38, sur la superficie totale de la surface externe 32 de la coque principale 30, est supérieur compris entre 40% et 45%.

La structure de la coque principale 30 a un taux de remplissage de l'ordre de 100%. Dans la variante représentée aux figures 4 et 5, la coque principale 30 comprend un ensemble d'arceaux 80, dans cet exemple précis au nombre de 15, s'étendant entre un sommet 90 de la coque principale 30, et une base annulaire transversale 100 passant par une partie des extrémités distales 85 des arceaux 80. La base annulaire 100 est dans un plan d'appui P1. Les extrémités distales 85 des arceaux 80 disposées au-dessus de la première ouverture transverse 50 ne sont pas dans le plan d'appui P1, mais distantes d'une hauteur h1 maximum dudit plan P1, pour la disposition d'au moins un pédicule vasculaire.

La coque principale 30 comprend des sections transversales 110, notamment au moins partiellement annulaires, s'étendant entre deux arceaux adjacents 80. La coque principale comprend plusieurs sections transversales 110 s'étendant entre des arceaux voisins, lesdites sections transversales 110 étant distantes les unes des autres. La coque principale 30 peut comprendre également un ensemble d'ouvertures traversantes 120 disposées au sommet 90.

Le dispositif 20 comprend également un fond principal 150 formant une partie postérieure 44 de la chambre de traitement 40, la coque principale 30 formant au moins une partie antérieure 42 de la chambre de traitement 40.

Le dispositif 20 comprend une coque intermédiaire 200 ayant une cloison supérieure de réception 210, subdivisant la chambre de traitement 40 entre une chambre supérieure de traitement 46 et une chambre inférieure de traitement 48. Chacune desdites chambres de traitement supérieure et inférieure 46,48 étant configurées pour recevoir au moins un pédicule vasculaire par l'intermédiaire des première et seconde ouvertures traversantes et transverses 50,60 pour la chambre de traitement supérieur 46 et par l'intermédiaire des première et secondes ouvertures traversantes et transverses 220 et 230 pour la chambre de traitement inférieure 48.

La cloison supérieure de réception 210 est configurée en sorte que lorsqu'elle est disposée dans la chambre de traitement 40, elle est espacée du fond principal 150 d'une distance d1 supérieur à 0 mm, par exemple de l'ordre de 2 mm à 8 mm, afin de délimiter au moins en partie la chambre de traitement inférieure 48. La coque intermédiaire 200 comprend des ouvertures traversantes 240 ménagées dans sa cloison latérale 250 et débouchant à la fois sur les faces interne 222 et externe 224 de ladite cloison latérale 250. La cloison latérale 250 se projette de la face inférieure de la cloison supérieure de réception 210 dans la chambre de traitement inférieure 48 et vers le fond principal 150.

La cloison latérale 250 a ainsi une hauteur h2 de l'ordre de la distance d1.

Dans un mode de réalisation, le rapport de la superficie totale des ouvertures traversantes 240 débouchant sur la face interne ou externe de la cloison latérale 250, sur la superficie totale de la face interne ou externe de la cloison latérale 250, est du même ordre que celui développé ci-dessus concernant les ouvertures traversantes 38 dans la coque principale 30.

Dans un mode de réalisation, le bord périphérique 31 de la coque principale 30 comprend une ou plusieurs projection(s) (ou bord(s) annulaire(s) inférieur(s)) 33 configurées pour coopérer avec une zone de réception 260 en creux, tel un logement, sensiblement annulaire, disposée sur le pourtour de la paroi supérieur de réception 210, pour la solidarisation de manière réversible entre la coque principale 30 et la coque intermédiaire 200.

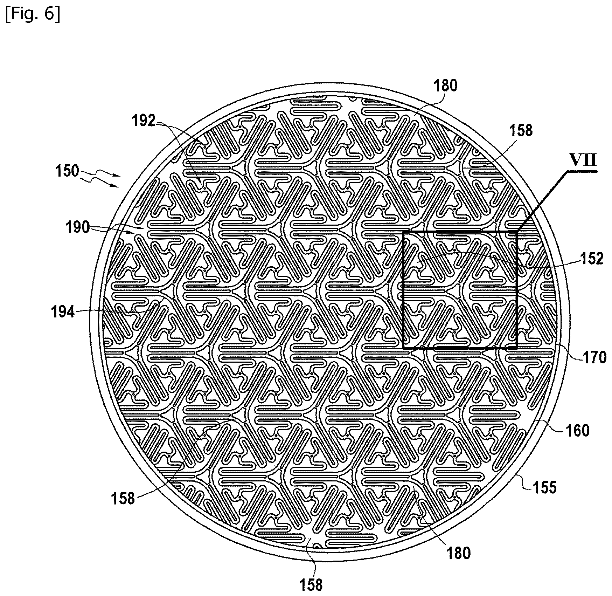

Dans un mode de réalisation, la cloison latérale 250 de la coque intermédiaire 200 comprend une ou plusieurs projection(s) (ou bord(s) annulaire(s) inférieur(s)) 270 configurées pour coopérer avec une zone de réception 160, tel un logement, sensiblement annulaire, représentée à la figure 6 et disposée sur le pourtour périphérique du fond principal 150, pour la solidarisation de manière réversible entre la coque intermédiaire 200 et le fond principal 150.

Cette zone de réception 160 est délimitée partiellement d'une part entre le rebord périphérique 170 se projetant d'une face supérieure 180 du fond principal 150 vers la chambre de traitement 40, en particulier vers la chambre de traitement inférieure 48, en particulier en retrait du bord externe 155 du fond principal 150, et d'autre part le bord externe 155.

Dans un mode de réalisation, le fond principal 150 représenté sur la figure 6 est sensiblement plane, et comprend selon sa face d'appui 152 des ouvertures traversantes 158 définissant un ensemble de premiers motifs 190, répétés sur la surface d'appui 152, liés les uns aux autres par des premières structures de liaison 192 et des secondes structures de liaison 194, également répétées sur la surface d'appui 152. Le premier motif 190 comprend quatre segments 191 parallèles entre eux et espacés les uns des autres par des ouvertures traversantes 158. Dans cet exemple précis, pour un même premier motif 190, deux segments 191 centraux sont solidarisés à une seconde structure de liaison 194 tandis qu'un premier segment externe 191 est solidarisé à une première structure de liaison 192, différente d'une autre première structure de liaison 192 à laquelle est solidarisé un second segment externe 191. Dans cet exemple précis, les premières structures de liaison 192, et les secondes structures de liaison 194, sont solidarisées, chacune, à trois premiers motifs 190. Les premières structures de liaison 192 ont des formes différentes des secondes structures de liaison 194.

Le fond principale 150 est ainsi très souple selon sa surface d'appui 152 tout en étant poreux. Dans cet exemple précis, la surface d'appui de la paroi supérieure de réception 210 est similaire à la surface d'appui 152.

L'agencement du fond principal 150 peut être également appliqué à la paroi supérieure de réception 260.

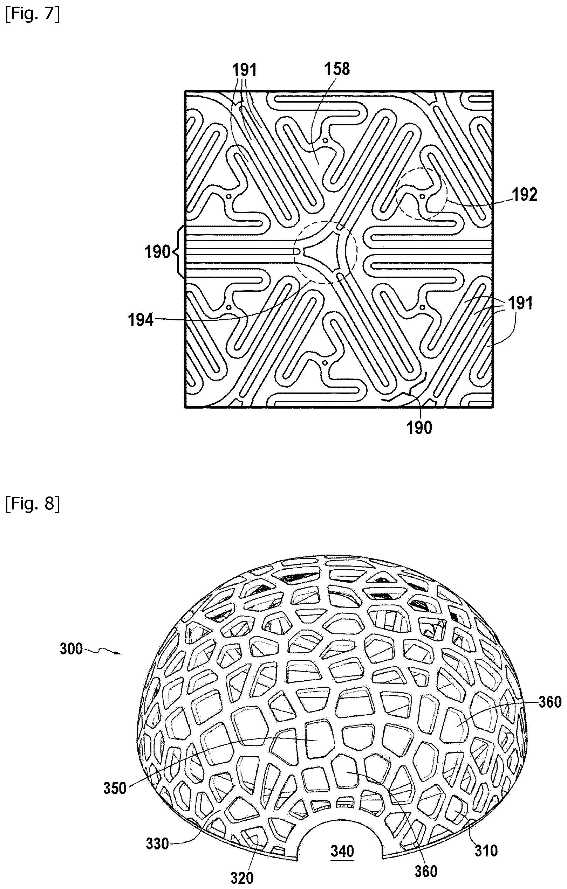

La coque principale 12 ou 30 peut être également remplacée par la coque principale 300 représentée à la figure 8. La coque principale 300 comprend des ouvertures traversantes 310 qui sont des polyèdres de Voronoï. La coque principale 300 comprend deux ouvertures traversantes latérales 340 débouchant dans la chambre de traitement 360, pour le placement d'au moins un pédicule vasculaire.

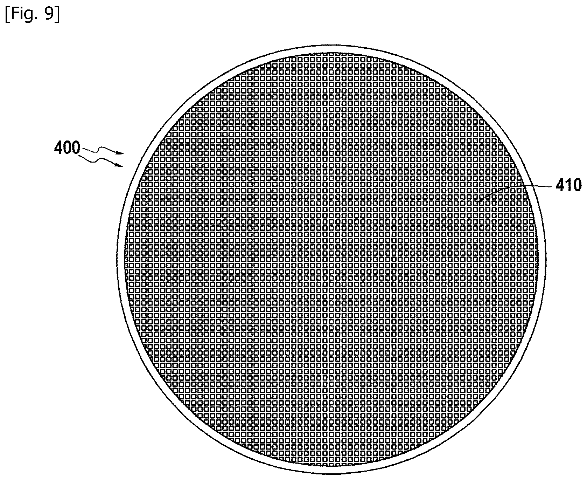

Le fond principal 150 ou 14 peut être également remplacée par le fond principal 400 représenté à la figure 9, différent du fond principal 150 ou 14, de par sa face d'appui 410 ne comprenant pas les premiers motifs, et premières et secondes structures de liaison mais de multiples ouvertures traversantes, notamment sensiblement parallélépipédiques. Cette surface d'appui 410 est également très souple et peut se déformer notamment dans sa région centrale mais pas autant que la face d'appui 152.

En fonctionnement, le dispositif implantable est sélectionné en fonction du volume de la chambre de traitement désiré. Si le volume est important, par exemple supérieur à 400-500 cm3, une coque principale, par exemple l'une des coques 10, 30 ou 300, est associée avec une coque intermédiaire, par exemple la coque intermédiaire 200, et un fond principal, par exemple le fond principal 14, 150 ou 400. Si le volume de la chambre de traitement désiré est inférieur à 400-500 cm3, une coque principale, par exemple l'une des coques 10, 30 ou 300, est associée directement avec un fond principal, par exemple le fond principal 14, 150 ou 400. Puis un ensemble comprenant au moins une couche poreuse support associée avec au moins une couche de cellules choisies parmi les adipocytes est disposé soit dans chacune des chambres de traitement supérieure et inférieure, ou dans la seule chambre de traitement. Au moins un pédicule vasculaire est associé avec un ensemble pour l'alimentation et la croissance du tissu mou.

La porosité de chacun des dispositifs implantables selon l'invention liée aux ouvertures traversantes ménagées dans la/les paroi(s) de la ou des coque(s) principale et/ou intermédiaire et/ou du fond principal favorise la croissance du tissu mou par la circulation homogène de liquide séreux. De plus, les surfaces d'appui sont souples et poreuses de par les ouvertures traversantes qu'elles comprennent favorisant ainsi la circulation de liquide séreux mais également la mise en contact des ensembles entre-eux disposés dans des chambres de traitement supérieure et inférieure séparées, et/ou avec la zone d'implantation s'agissant du fond principal. Enfin, la masse des dispositifs est optimisée tout en offrant des performances mécaniques satisfaisantes grâce à la mise en œuvre d'un (co)polymère très souple tel que défini dans le présent texte.

## Revendications

1. Dispositif implantable (10,20), notamment mammaire, comprenant une coque principale (12,30,300) et une chambre de traitement (5,40,360) d'au moins un tissu mou, ledit dispositif est en outre configuré pour recevoir au moins un pédiculaire vasculaire, la coque principale (12,30,300) comprend un ensemble d'ouvertures traversantes (8,16,38,50,60,120,310) et est biorésorbable, **caractérisé en ce que** ladite coque principale (12,30,300) comprend au moins un (co)polymère biorésorbable ayant un allongement à rupture supérieur ou égal à 200%, **en ce que** ledit (co)polymère est un polymère de ε-caprolactone et d'au moins une unité de répétition différente du ε-caprolactone issue de l'acide lactique et/ou de l'acide glycolique, **en ce que** la coque principale comprend une surface externe et une surface interne orientée en regard de la chambre de traitement, les surfaces interne et externe sont sensiblement opposées, **en ce que** lesdites ouvertures traversantes débouchent sur les surfaces externe et interne de la coque principale et s'étendent entre lesdites surfaces externe et interne, **en ce que** ledit au moins un (co)polymère comprend des unités de répétition du ε-caprolactone dont la fraction molaire dans ledit (co)polymère est supérieure ou égale à 20% et inférieure ou égale à 50%.

2. Dispositif implantable (10,20) selon la revendication 1, **caractérisé en ce que** ledit copolymère biorésorbable est un copolymère de ε-caprolactone et de lactide de forme L ou D ou L,D.

3. Dispositif implantable (10,20) selon l'une ou l'autre des revendications 1 et 2, **caractérisé en ce que** la coque principale (12,30,300) a une surface externe (12a,32), et **en ce que** le rapport de, la superficie totale (mm²) des ouvertures traversantes (8,16,38,50,60,120,310), sur la superficie totale de la surface externe (12a,32) de la coque principale (12,30,300), est supérieur ou égal à 35%, de préférence inférieur ou égal à 60%.

4. Dispositif implantable (10,20) selon l'une quelconque des revendications 1 à 3, **caractérisé en ce que** la coque principale (12,30,300) a une surface externe (12a,32), et **en ce que** le rapport de, la superficie de la surface externe pleine de la coque principale (12,30,300), sur la superficie totale de la surface externe (12a,32) de la coque principale (12,30,300), est supérieur ou égal à 40%, de préférence supérieur ou égal à 50%, en particulier inférieur ou égal à 70%.

5. Dispositif implantable (10,20) selon l'une quelconque des revendications 1 à 4, **caractérisé en ce que** la coque principale a un squelette ayant un taux de remplissage supérieur ou égal à 70%.

6. Dispositif implantable (10,20) selon l'une quelconque des revendications 1 à 5, **caractérisé en ce qu'**il comprend un fond principal (14,150,400) ayant des ouvertures traversantes (16,19,158) et formant une partie postérieure (44) de la chambre de traitement (5,40,360).

7. Dispositif implantable (20) selon l'une quelconque des revendications 1 à 6, **caractérisé en ce qu'**il comprend une coque intermédiaire (200) comprenant des ouvertures traversantes (240,158) et une cloison supérieure de réception (210) subdivisant la chambre de traitement (40) entre une chambre supérieure de traitement (46) et une chambre inférieure de traitement (48), ledit dispositif étant configuré pour que chacune desdites chambres de traitement supérieure (46) et inférieure (48) soit apte à recevoir au moins un pédicule vasculaire.

8. Dispositif implantable (20) selon les revendications 6 et 7, **caractérisé en ce que** ladite cloison supérieure de réception (210) est espacée du fond principal (150,400) d'une distance d (d1) supérieure à 0 mm afin de délimiter au moins en partie la chambre de traitement inférieure (48).

9. Dispositif (20) selon l'une ou l'autre des revendications 7 et 8, **caractérisé en ce que** la coque intermédiaire (200) comprend une cloison latérale (250) se projetant d'une face inférieure de la cloison supérieure de réception (210), en particulier dans la chambre de traitement inférieure (48).

10. Dispositif (10,20) selon l'une quelconque des revendications 6 et 8, **caractérisé en ce que** le fond principal (14,150) comprend un ou plusieurs logements (21,160), ouvert(s) vers la chambre de traitement (5,40), et configuré(s) pour recevoir, notamment par emboîtement, une partie ou des parties d'un bord inférieur (13,33,270), notamment d'un bord annulaire inférieur (13,33,270), de la coque principale (12,30) ou de la coque intermédiaire (200).

11. Dispositif (10) selon les revendications 6 et 10, **caractérisé en ce que** le fond principal (14) comprend :
- un rebord externe (15), continu ou discontinu, se projetant d'une face interne (14b) du fond principal (14) dans la chambre de traitement (5), et
- une ou plusieurs portions de paroi vertical (18) se projetant de la face interne (14b) du fond principal dans la chambre de traitement (5), et délimitant au moins en partie avec ledit rebord externe (15), le ou lesdits logement(s) (21).

12. Dispositif (10) selon l'une ou l'autre des revendications 10 et 11, **caractérisé en ce que** la coque principale (12) ou la coque intermédiaire comprend un bord externe inférieur (13a), et un bord interne inférieur (13b) en retrait dudit bord externe inférieur (13a).

13. Dispositif (10) selon les revendications 11 et 12, **caractérisé en ce que** le bord externe inférieur (13b) vient en appui sur le rebord externe (15) du fond principal (14).

14. Dispositif (10) selon l'une quelconque des revendications 6, 8, et 10 à 13, **caractérisé en ce que** la coque principale (12) comprend une ou des ouvertures de solidarisation (8), et le fond principal (14) comprend une ou des ouvertures de solidarisation (19), lesdites ouvertures de solidarisation (8,19) étant configurées en sorte de permettre le passage d'au moins un organe de solidarisation à travers une ouverture de solidarisation (8) de la coque principale (12) et une ouverture de solidarisation (9) du fond principal (14).

15. Dispositif implantable selon l'une quelconque des revendications 1 à 14, **caractérisé en ce qu'**au moins une partie des ouvertures traversantes (360) de la coque principale (300) sont des polyèdres de Voronoï.

16. Dispositif implantable (20) selon l'une quelconque des revendications 1 à 14, **caractérisé en ce que** la coque principale (30) comprend au moins deux arceaux verticaux (80), et des sections transversales (110), notamment au moins partiellement annulaires, s'étendant entre lesdits arceaux verticaux (80) et en liaison avec ces derniers.

17. Dispositif implantable (20) selon l'une quelconque des revendications 6, 8, et 10 à 14, **caractérisé en ce que** le fond principal (150) comprend des ouvertures traversantes (158) agencées pour définir plusieurs premiers motifs (190) liés les uns aux autres par des premières structures de liaison (192).

18. Dispositif (20) selon la revendication 17, **caractérisé en ce que** les premiers motifs (190) comprennent un ou plusieurs segment(s) (191), notamment au moins trois segments et au plus six segments, au moins l'un du ou des segment(s) (191) de chaque premier motif (190) est solidarisé à une première structure de liaison (192).

19. Dispositif (20) selon l'une ou l'autre des revendications 17 et 18, **caractérisé en ce que** les premières structures de liaison (192) sont reliées à au moins trois premiers motifs distincts (190).

20. Dispositif (20) selon l'une ou l'autre des revendications 18 et 19, **caractérisé en ce que** le fond principal (150) comprend des secondes structures de liaison (194), différentes des premières structures de liaison (192), chacune des secondes structures de liaison (194) est reliée à au moins deux segments (191) d'un même premier motif (190).

21. Dispositif (20) selon la revendication 20, **caractérisé en ce qu'**au moins l'une des seconde(s) structure(s) de liaison (194) est/sont reliée(s) à au moins trois premiers motifs distincts (190).

22. Procédé de fabrication d'un dispositif implantable (10,20) selon l'une quelconque des revendications 1 à 21, **caractérisé en ce qu'**il comprend une étape de fabrication additive ou soustractive, de préférence additive, de ladite coque principale (12,30,300), avec au moins un fil comprenant ledit (co)polymère ayant un allongement à la rupture supérieure ou égale à 200%, **en ce que** ledit (co)polymère est un polymère de ε-caprolactone et d'au moins une unité de répétition différente du ε-caprolactone issue de l'acide lactique et/ou de l'acide glycolique, **en ce que** ledit (co)polymère comprend des unités de répétition du ε-caprolactone dont la fraction molaire dans ledit (co)polymère est supérieure ou égale à 20% et inférieure ou égale à 50%.

## Patentansprüche

1. Implantierbare Vorrichtung (10, 20), insbesondere für die Brust, umfassend eine Hauptschale (12, 30, 300) und eine Behandlungskammer (5, 40, 360) für mindestens ein Weichgewebe, wobei die Vorrichtung ferner konfiguriert ist, um mindestens einen Gefäßstiel aufzunehmen, wobei die Hauptschale (12, 30, 300) eine Anordnung von Durchgangsöffnungen (8, 16, 38, 50, 60, 120, 310) umfasst und bioresorbierbar ist, **dadurch gekennzeichnet, dass** die Hauptschale (12, 30, 300) mindestens ein bioresorbierbares (Co)Polymer umfasst, das eine Bruchdehnung von größer als oder gleich wie 200 % umfasst, dass das (Co)Polymer ein Polymer aus E-Caprolacton und mindestens einer von E-Caprolacton verschiedenen Wiederholungseinheit aus Milchsäure und/oder Glykolsäure ist, dass die Hauptschale eine Außenfläche und eine der Behandlungskammer zugewandte Innenfläche umfasst, wobei die Innen- und die Außenfläche im Wesentlichen einander gegenüber sind, dass die Durchgangsöffnungen an der Außen- und der Innenfläche der Hauptschale münden und sich zwischen der Außen- und der Innenfläche erstrecken, und dass das mindestens eine (Co)Polymer Wiederholungseinheiten von E-Caprolacton umfasst, deren Mol-Fraktion in dem (Co)Polymer größer als oder gleich wie 20 % und kleiner als oder gleich wie 50 % ist.

2. Implantierbare Vorrichtung (10, 20) nach Anspruch 1, **dadurch gekennzeichnet, dass** das bioresorbierbare Copolymer ein Copolymer aus ε-Caprolacton und Lactid in L-, D- oder L,D-Form ist.

3. Implantierbare Vorrichtung (10, 20) nach einem der Ansprüche 1 und 2, **dadurch gekennzeichnet, dass** die Hauptschale (12, 30, 300) eine Außenfläche (12a, 32) aufweist, und dass das Verhältnis der Gesamtfläche (mm²) der Durchgangsöffnungen (8, 16, 38, 50, 60, 120, 310) zu der Gesamtfläche der Außenfläche (12a, 32) der Hauptschale (12, 30, 300) größer als oder gleich wie 35 %, vorzugsweise kleiner als oder gleich wie 60 %, ist.

4. Implantierbare Vorrichtung (10, 20) nach einem der Ansprüche 1 bis 3, **dadurch gekennzeichnet, dass** die Hauptschale (12, 30, 300) eine Außenfläche (12a, 32) aufweist, und dass das Verhältnis der Fläche der vollen Außenfläche der Hauptschale (12, 30, 300) zu der Gesamtfläche der Außenfläche (12a, 32) der Hauptschale (12, 30, 300) größer als oder gleich wie 40 %, vorzugsweise größer als oder gleich wie 50 %, insbesondere kleiner als oder gleich wie 70 %, ist.

5. Implantierbare Vorrichtung (10, 20) nach einem der Ansprüche 1 bis 4, **dadurch gekennzeichnet, dass** die Hauptschale ein Gerüst mit einem Füllgrad von größer als oder gleich wie 70 % aufweist.

6. Implantierbare Vorrichtung (10, 20) nach einem der Ansprüche 1 bis 5, **dadurch gekennzeichnet, dass** sie einen Hauptboden (14, 150, 400) umfasst, der Durchgangsöffnungen (16, 19, 158) aufweist und einen hinteren Teil (44) der Behandlungskammer (5, 40, 360) bildet.

7. Implantierbare Vorrichtung (20) nach einem der Ansprüche 1 bis 6, **dadurch gekennzeichnet, dass** sie eine Zwischenschale (200) umfasst, umfassend Durchgangsöffnungen (240, 158) und eine obere Aufnahmetrennwand (210), die die Behandlungskammer (40) in eine obere Behandlungskammer (46) und eine untere Behandlungskammer (48) unterteilt, wobei die Vorrichtung konfiguriert ist, damit jede von der oberen (46) und der unteren (48) Behandlungskammer geeignet ist, mindestens einen Gefäßstiel aufzunehmen.

8. Implantierbare Vorrichtung (20) nach den Ansprüchen 6 und 7, **dadurch gekennzeichnet, dass** die obere n de (210) vom Hauptboden (150, 400) um einen Abstand d (d1) von größer als 0 mm beabstandet ist, um die untere Behandlungskammer (48) zumindest teilweise zu begrenzen.

9. Vorrichtung (20) nach einem der Ansprüche 7 und 8, **dadurch gekennzeichnet, dass** die Zwischenschale (200) eine seitliche Trennwand (250) umfasst, die von einer Unterseite der oberen Aufnahmetrennwand (210) insbesondere in die untere Behandlungskammer (48) hervorsteht.

10. Vorrichtung (10, 20) nach einem der Ansprüche 6 und 8, **dadurch gekennzeichnet, dass** der Hauptboden (14, 150) eine oder mehrere Aufnahmen (21, 160) umfasst, die zu der Behandlungskammer (5, 40) hin offen und konfiguriert sind, um insbesondere durch Ineinanderstecken, einen Teil oder Teile eines unteren Rands (13, 33, 270), insbesondere eines unteren Ringrands (13, 33, 270), der Hauptschale (12, 30) oder der Zwischenschale (200) aufzunehmen.

11. Vorrichtung (10) nach den Ansprüchen 6 und 10, **dadurch gekennzeichnet, dass** der Hauptboden (14) Folgendes umfasst:
- einen kontinuierlichen oder diskontinuierlichen äußeren Rand (15), der von einer Innenfläche (14b) des Hauptbodens (14) in die Behandlungskammer (5) hervorsteht, und
- einen oder mehrere vertikale Wandabschnitte (18), die von der Innenfläche (14b) des Hauptbodens in die Behandlungskammer (5) hervorstehen und zusammen mit dem äußeren Rand (15) die Aufnahme(n) (21) zumindest teilweise begrenzen.

12. Vorrichtung (10) nach einem der Ansprüche 10 und 11, **dadurch gekennzeichnet, dass** die Hauptschale (12) oder die Zwischenschale einen unteren äußeren Rand (13a) und einen gegenüber dem unteren äußeren Rand (13a) zurückgesetzten unteren inneren Rand (13b) umfasst.

13. Vorrichtung (10) nach den Ansprüchen 11 und 12, **dadurch gekennzeichnet, dass** der untere äußere Rand (13b) an dem äußeren Rand (15) des Hauptbodens (14) anliegt.

14. Vorrichtung (10) nach einem der Ansprüche 6, 8 und 10 bis 13, **dadurch gekennzeichnet, dass** die Hauptschale (12) eine oder mehrere Befestigungsöffnungen (8) umfasst und der Hauptboden (14) eine oder mehrere Befestigungsöffnungen (19) umfasst, wobei die Befestigungsöffnungen (8, 19) konfiguriert sind, um einen Durchgang mindestens eines Befestigungsorgans durch eine Befestigungsöffnung (8) der Hauptschale (12) und eine Befestigungsöffnung (19) des Hauptbodens (14) zu ermöglichen.

15. Implantierbare Vorrichtung nach einem der Ansprüche 1 bis 14, **dadurch gekennzeichnet, dass** mindestens ein Teil der Durchgangsöffnungen (360) der Hauptschale (300) Voronoi-Polyeder sind.

16. Implantierbare Vorrichtung (20) nach einem der Ansprüche 1 bis 14, **dadurch gekennzeichnet, dass** die Hauptschale (30) mindestens zwei vertikale Bögen (80) und Querschnitte (110), insbesondere zumindest teilweise ringförmige Querschnitte, umfasst, die sich zwischen den vertikalen Bögen (80) erstrecken und damit verbunden sind.

17. Implantierbare Vorrichtung (20) nach einem der Ansprüche 6, 8 und 10 bis 14, **dadurch gekennzeichnet, dass** der Hauptboden (150) Durchgangsöffnungen (158) umfasst, die ausgebildet sind, um mehrere erste Muster (190) definieren, die durch erste Verbindungsstrukturen (192) miteinander verbunden sind.

18. Vorrichtung (20) nach Anspruch 17, **dadurch gekennzeichnet, dass** die ersten Muster (190) ein oder mehrere Segment(e) (191) umfassen, insbesondere mindestens drei Segmente und höchstens sechs Segmente, wobei mindestens eines des oder der Segmente (191) von jedem ersten Muster (190) mit einer ersten Verbindungsstruktur (192) verbunden ist.

19. Vorrichtung (20) nach einem der Ansprüche 17 und 18, **dadurch gekennzeichnet, dass** die ersten Verbindungsstrukturen (192) mit mindestens drei verschiedenen ersten Mustern (190) verbunden sind.

20. Vorrichtung (20) nach einem der Ansprüche 18 und 19, **dadurch gekennzeichnet, dass** der Hauptboden (150) zweite Verbindungsstrukturen (194) umfasst, die sich von den ersten Verbindungsstrukturen (192) unterscheiden, wobei jede der zweiten Verbindungsstrukturen (194) mit mindestens zwei Segmenten (191) ein und desselben ersten Musters (190) verbunden ist.

21. Vorrichtung (20) nach Anspruch 20, **dadurch gekennzeichnet, dass** mindestens eine der zweiten Verbindungsstruktur(en) (194) mit mindestens drei verschiedenen ersten Mustern (190) verbunden ist/sind.

22. Verfahren zur Herstellung einer implantierbaren Vorrichtung (10, 20) nach einem der Ansprüche 1 bis 21, **dadurch gekennzeichnet, dass** es einen Schritt einer additiven oder subtraktiven, vorzugsweise additiven, Fertigung der Hauptschale (12, 30, 300) mit mindestens einem Faden umfasst, der das (Co)Polymer mit einer Bruchdehnung von größer als oder gleich wie 200% umfasst, dass das (Co)Polymer ein Polymer aus E-Caprolacton und mindestens einer von E-Caprolacton verschiedenen Wiederholungseinheit aus Milchsäure und/oder Glykolsäure ist, und dass das (Co)Polymer Wiederholungseinheiten von E-Caprolacton umfasst, deren Mol-Fraktion in dem (Co)Polymer größer als oder gleich wie 20 % und kleiner als oder gleich wie 50 % ist.

## Claims

1. An implantable device (10, 20), in particular an implantable breast device, comprising a main shell (12, 30, 300) and a chamber (5, 40, 360) for treating at least one soft tissue, said device is further configured to receive at least one vascular pedicle, the main shell (12, 30, 300) comprising a set of through-openings (8, 16, 38, 50, 60, 120, 310) and is bioresorbable, **characterised in that** said main shell (12, 30, 300) comprises at least one bioresorbable (co)polymer having an elongation at break greater than or equal to 200%, **in that** said (co)polymer is a polymer of ε-caprolactone and at least one repeat unit different from ε-caprolactone derived from lactic acid and/or glycolic acid, **in that** the main shell comprises an outer surface and an inner surface oriented facing the treatment chamber, the inner surface is substantially opposite the outer surface, **in that** the through-openings emerge on the outer and inner surfaces of the main shell and extend between said outer and inner surfaces, **in that** said at least one bioresorbable (co)polymer comprises ε-caprolactone repeat units the molar fraction of which is higher than or equal to 20% and less than or equal to 50%.

2. The implantable device (10, 20) according to claim 1, **characterised in that** said bioresorbable copolymer is a copolymer of ε-caprolactone and L-, D- or DL-lactide.

3. The implantable device (10, 20) according to one or other of claims 1 and 2, **characterised in that** the main shell (12, 30, 300) has an outer surface (12a, 32), and **in that** the ratio of, the total surface area (mm²) of the through-openings (8, 16, 38, 50, 60, 120, 310), over the total surface area of the outer surface (12a, 32) of the main shell (12, 30, 300), is greater than or equal to 35%, preferably less than or equal to 60%.

4. The implantable device (10, 20) according to any one of claims 1 to 3, **characterised in that** the main shell (12, 30, 300) has an outer surface (12a, 32), and **in that** the ratio of, the surface area of the solid outer surface of the main shell (12, 30, 300), over the total surface area of the outer surface (12a, 32) of the main shell (12, 30, 300), is greater than or equal to 40%, preferably greater than or equal to 50%, in particular less than or equal to 70%.

5. The implantable device (10, 20) according to any one of claims 1 to 4, **characterised in that** the main shell has a skeleton having a fill ratio greater than or equal to 70%.

6. The implantable device (10, 20) according to any one of claims 1 to 5, **characterised in that** it comprises a main bottom (14, 150, 400) having through-openings (16, 19, 158) and forming a rear portion (44) of the treatment chamber (5, 40, 360).

7. The implantable device (20) according to any one of claims 1 to 6, **characterised in that** it comprises an intermediate shell (200) comprising through-openings (240, 158) and an upper receiving partition (210) subdividing the treatment chamber (40) into an upper treatment chamber (46) and a lower treatment chamber (48), said device being configured so that each of said upper (46) and lower (48) treatment chambers is able to receive at least one vascular pedicle.

8. The implantable device (20) according to claims 6 and 7, **characterised in that** said upper receiving partition (210) is spaced apart from the main bottom (150, 400) by a distance d (d1) greater than 0 mm in order to at least partially delimit the lower treatment chamber (48).

9. The device (20) according to one or other of claims 7 and 8, **characterised in that** the intermediate shell (200) comprises a lateral partition (250) projecting from a lower face of the upper receiving partition (210), in particular into the lower treatment chamber (48).

10. The device (10, 20) according to any one of claims 6 and 8, **characterised in that** the main bottom (14, 150) comprises one or more recesses (21, 160), open towards the treatment chamber (5, 40), and configured to receive, in particular by interlocking, a portion or portions of a lower edge (13, 33, 270), in particular an annular lower edge (13, 33, 270) of the main shell (12, 30) or of the intermediate shell (200).

11. The device (10) according to claims 6 and 10, **characterised in that** the main bottom (14) comprises:
- a continuous or discontinuous outer rim (15), projecting from an inner face (14b) of the main bottom (14) into the treatment chamber (5), and
- one or more portions of vertical wall (18) projecting from the inner face (14b) of the main bottom into the treatment chamber (5), and at least partially limiting, with said outer rim (15), said recess or recesses (21).

12. The device (10) according to one or other of claims 10 and 11, **characterised in that** the main shell (12) or the intermediate shell comprises an outer lower edge (13a), and an inner lower edge (13b) set back from said outer lower edge (13a).

13. The device (10) according to claims 11 and 12, **characterised in that** the outer lower edge (13b) abuts on the outer rim (15) of the main bottom (14).

14. The device (10) according to any one of claims 6, 8, and 10 to 13, **characterised in that** the main shell (12) comprises one or more securing openings (8), and the main bottom (14) comprises one or more securing openings (19), said securing openings (8, 19) being configured so as to allow the passage of at least one securing member through a securing opening (8) of the main shell (12) and a securing opening (9) of the main bottom (14).

15. The implantable device according to any one of claims 1 to 14, **characterised in that** at least one portion of the through-openings (360) of the main shell (300) are Voronoi polyhedra.

16. The implantable device (20) according to any one of claims 1 to 14, **characterised in that** the main shell (30) comprises at least two vertical arches (80), and transverse sections (110), in particular at least partially annular, extending between said vertical arches (80) and connected thereto.

17. The implantable device (20) according to any one of claims 6, 8, and 10 to 14, **characterised in that** the main bottom (150) comprises through-openings (158) arranged to define a plurality of first patterns (190) connected to one another by first connection structures (192).

18. The device (20) according to claim 17, **characterised in that** the first patterns (190) comprise one or more segments (191), in particular at least three segments and at most six segments, at least one or more segments (191) of each first pattern (190) being secured to a first connection structure (192).

19. The device (20) according to one or other of claims 17 and 18, **characterised in that** the first connection structures (192) are connected to at least three distinct first patterns (190).

20. The device (20) according to one or other of claims 18 and 19, **characterised in that** the main bottom (150) comprises second connection structures (194), different from the first connection structures (192), each of the second connection structures (194) is connected to at least two segments (191) of a same first pattern (190).

21. The device (20) according to claim 20, **characterised in that** at least one of the second connection structures (194) is connected to at least three distinct first patterns (190).

22. A method for manufacturing an implantable device (10, 20) according to any one of claims 1 to 21, **characterised in that** it comprises a step of additive or subtractive manufacturing, preferably additive, of said main shell (12, 30, 300), with at least one yarn comprising said (co)polymer having an elongation at break greater than or equal to 200%, **in that** said (co)polymer is a polymer of ε-caprolactone and at least one repeat unit different from ε-caprolactone derived from lactic acid and/or glycolic acid, **in that** at least one bioresorbable (co)polymer comprises ε-caprolactone repeat units the molar fraction of which is higher than or equal to 20% and less than or equal to 50%.
